# EUROPEAN PATENT SPECIFICATION

(11) **EP 1 229 865 B1**
(45) Date of publication and mention of the grant of the patent: **17.11.2010**
(21) Application number: 99971225.0
(22) Date of filing: 22.12.1999
(51) Int. Cl.: A61F 2/06, A61F 2/24, A61L 27/36

(54) **ENDOVASCULAR TREATMENT FOR CHRONIC VENOUS INSUFFICIENCY**
ENDOVASKULÄRE BEHANDLUNG CHRONISCHER VENÖSER INSUFFIZIENZ
TRAITEMENT ENDOVASCULAIRE DES INSUFFISANCES VEINEUSES CHRONIQUES

(30) Priority: 10.09.1999 US 153367 P
(43) Date of publication of application: 14.08.2002
(73) Proprietor: Cook Incorporated, Bloomington, Indiana 47404 (US)
(72) Inventor: Thorpe, Patricia Ellen, Iowa City Iowa 52245 (US); Osse, Francisco J, Alto da Lapa Sao Paulo-SP 05084-010 (BR)
(74) Representative: Waldren, Robin Michael
(86) International application number: PCT/US1999/030808
(87) International publication number: WO 2001/019285

(56) References cited:
- EP-A- 0 808 614
- EP-A- 0 850 607
- WO-A-98/25549
- US-A- 5 116 564
- US-A- 5 123 919
- US-A- 5 480 424
- US-A- 5 500 014
- US-A- 5 607 465
- US-A- 5 851 232
- US-A- 5 855 597
- US-A- 5 855 601
- US-A- 6 027 525

## Description

### Field of the Invention

The present invention relates to venous valve replacement and, in particular, to replacement venous valves to lower extremities and a therapeutic method of treating venous circulatory disorders.

### Background of the Invention

Chronic venous insufficiency (CVI) of the lower extremities is a common condition that is considered a serious public health and socioeconomic problem. In the United States, approximately two million workdays are lost each year, and over 2 million new cases of venous thrombosis are recorded each year. About 800,000 new cases of venous insufficiency syndrome will also be recorded annually. Ambulatory care costs of about $2,000, per patient, per month contribute to the estimated U.S. cost of $16,000,000 per month for the treatment of venous stasis ulcers related to CVI.

It is estimated that greater than 3 % of the Medicare population is afflicted by a degree of CVI manifested as non-healing ulcers. Studies have indicated that about 40% of seriously affected individuals cannot work or even leave the house except to obtain medical care. It is estimated that 0.2% of the American work force is afflicted with CVI.

Chronic venous insufficiency arises from long duration venous hypertension caused by valvular insufficiency and/or venous obstruction secondary to venous thrombosis. Other primary causes of CVI include varicosities of long duration, venous hypoplasia and arteriovenous fistula. The signs and symptoms of CVI have been used to classify the degree of severity of the disease and reporting standards have been published. Studies demonstrate that deterioration of venous hemodynamic status correlates with disease severity. Venous reflux, measured by ultrasound studies, is the method of choice of initial evaluation of patients with pain and/or swelling in the lower extremities. In most serious cases of CVI, venous stasis ulcers are indicative of incompetent venous valves in all systems, including superficial, common, deep and communicating veins. This global involvement affects at least 30% of all cases. Standard principles of treatment are directed at elimination of venous reflux. Based on this observation, therapeutic intervention is best determined by evaluating the extent of valvula incompetence, and the anatomical distribution of reflux. Valvular incompetence, a major component of venous hypertension, is present in about 60% of patients with a clinical diagnosis of CVI.

Endovascular valve replacement refers to a new concept and new technology in the treatment of valvular reflux. The concept involves percutaneous insertion of the prosthetic device under fluoroscopic guidance. The device can be advanced to the desired intravascular location using guide wires and catheters. Deployment at a selected site can be accomplished to correct valvular incompetence. Percutaneous placement of a new valve apparatus provides a less invasive solution compared to surgical transposition or open repair of a valve.

The modern concept of a stent was introduced in the 1960s. Subsequently, it has been successfully incorporated in the treatment of arterioral aneurysms and occlusive disease. The use of endovascular stents represents one of the most significant changes in the field of vascular surgery since the introduction of surgical graft techniques in the early 1950s.

Initially, the dominant interest of vascular specialists was application of stents in the arterial system. The venous system and venous disease were not considered an arena for stent application. The utilization of cndovascular treatment in venous disease was initially confined to the treatment of obstruction, in the pelvic veins [for CVT] as well as treatment of obstructed hemodialysis access grafts and decompression of portal hypertension (TIPS). Although these procedures enjoy widespread application, the actual number of patients involved ins relatively low compared to the number afflicted with CVI and related syndrome. Thus, the necessity for therapy using endovascular technology for the treatment of venous disease arose. The prevalence of CVI and the magnitude of its impact demand development of an effective alternative therapy.

EP 0 808 614 A2 discloses a stent having cylindrical radial elastic units and a cylindrical cover fixing member sheathed over the elastic units to fix the units in a cylindrical shape by connecting the elastic units. U.S. Patent No. 5,851,232 discloses a stent assembly having a cylindrical shell and a plurality of circular coils embedded within the cylindrical shell. The stent assembly is changeable between a first unexpanded state for placement within a body lumen and a second expanded state for holding the body lumen open once positioned therein. The plurality of coils are formed of cut rings that are staggered about the radius of the cylindrical shell to provide support for the shell throughout its circumference. The shell may include ridges extending from an outer wall thereof for engaging the inner wall and holding the stent assembly in place. U.S. Patent No. 5.855.60t is directed to an artificial heart valve and method and device for implanting the same. The disclosed valve includes a relatively rigid self-expanding stent member having a cylindrical shape and a flexible valve disposed therein. The valve includes a circular portion having a plurality of leaflets extending from the periphery toward the center of the valve. The diameter of the circular portion is substantially the same as the inside diameter of the stent member when the stent member is expanded. U.S. Patent No. 5,855,597 is related to a star-shaped stent and replacement valve for use in repairing a damaged cardiac valve. The stent is made by cutting a star-shaped member from a flat metal sheet and interconnecting the star-shaped members into a chain. PCT Application WO 98/25549 relates to artificial vascular valves. In particular, the invention is directed to the use of submucosal tissue to prepare tissue valve constructs and the use of those valve constructs to replace or repair damaged or diseased valves of the heart and the circulatory system of warm-blooded vertebrae. EP 0 850 607 shows a valve prothesis.

### Brief Description of the Drawings

Figure 1 is a schematic representation of a portion of a venous system.
Figure 2 is a schematic representation of a section view of a portion of a venous system at a closed venous valve.
Figure 3 is a schematic representation of a sectional view of a portion of a venous system.
Figure 4 is a schematic representation of a portion of a venous system.
Figure 5 is a schematic representation of a section view of a portion of a venous system at an open venous valve.
Figure 6 is a schematic representation of a section view of a portion of a venous system showing a deployment system for a devices of the invention.
Figure 7 is a schematic representation of a section view of a portion of a venous system showing a deployed device of the invention.
Figure 8 is a schematic view of one embodiment of the invention.
Figure 9 is a schematic view of one embodiment of the invention.
Figure 10 is a schematic view of one embodiment of the invention illustrating angular relationships of components.
Figure 11 is a top plan view taken along line 11-11 of Figure 9.
Figure 12 is a schematic elevation view of one embodiment of the invention.
Figure 13 is a schematic view of various valve material placement embodiments of the invention.
Figure 14 is a schematic view of a multiple stage embodiment of the invention.
Figure 15 is a side elevation view of a six strut dual stage embodiment of the invention.
Figure 16 is a side elevation view of a six strut dual stage truncated cone embodiment of the invention.
Figure 17 is a photo image of an embodiment of the invention in vivo.
Figure 18 is a photo image of an embodiment of the invention in vivo.
Figure 19 is a photo image of an embodiment of the invention in vivo.
Figure 20 is a photo image of an embodiment of the invention in vivo.
Figure 21 is a photo image of an embodiment of the invention in vivo.
Figure 22 is a photo image of an embodiment of the invention in vivo.
Figure 23 is a photo image of an embodiment of the invention in vivo.
Figure 24 is a perspective view of one embodiment of the invention.
Figure 25 is a flow diagram depicting one embodiment of the invention.
Figure 26 is a flow diagram depicting one embodiment of the invention.

### Summary Of The Invention

According to a first aspect of the present invention there is provided a replacement valve assembly which is configured for implantation within a venous lumen, the valve assembly including a self-expanding stent and a plurality of flexible members, each flexible member conformed to cooperate with at least one other flexible member to unidirectionally admit vascular fluid through the valve assembly and to prevent retrograde flow of the vascular fluid through the valve assembly, characterized in that the stent includes at least two stacks joined so that at least one end of each stack is directly interconnected in series to an end of another stack for allowing deployment of one stack prior to deployment of said another stack.

Preferably, there is also provided a method of making the replacement valve assembly defined above for implantation, the method comprising the steps of:
providing a flexible biocompatible material;
constructing a plurality of flexible members from the flexible material; and
disposing the flexible members in a tubular member having at least two stacks joined so that at least one end of each stack is directly interconnected in series to an end of another stack for allowing deployment of one stack prior to deployment of said another stack and joined so as to function as a directional flow valve.

The valve assembly comprises a plurality of flexible members, with each flexible member arranged to cooperate with at least one other flexible member to unidirectionally admit vascular fluid through the valve assembly. In one embodiment, at least a portion of one of the flexible members includes natural selera tissue. In other embodiments, the flexible members include at least a portion of either SIS or other known biocompatible material. Methods of manufacturing the flexible members and of assembling and delivering the assembly to the patient's venous system are also provided.

### Detailed Description of the Preferred Embodiments

Within the field of endovascular treatment, no previous technology has effectively combined a replacement valve and a stent in a percutaneously located assembly. Indeed, recognition of the need for such a device, system and method of employment has been lacking. Attempts at venous valve repair are not common. Indeed, minimally invasive repair or replacement procedure are quite uncommon. This is due, in part, to the poor availability of properly sized and properly designed prosthetic venous valves. United States Patent 5,500,014 has an excellent discussion of the different attempts to provide prosthetic venous valves. For the anatomy of venous valves, an excellent reference includes Venous Valves, by R.Gottlub and Ramey, published by Springer-Verlag, Austria, 1986.

The inventors have devised a device, system and method of deployment for a stent and valve assembly utilizing various materials having excellent cost, biocompatibility, and ease of use. In one embodiment, a stent is assembled having excellent length and stability characteristics, as well as an improved profile for ease of placement and automatic deployment at a deployment site. The assembly does not rely on placement at a previous valvular site but may be utilized either proximate or distal to the incompetent valve site due to the self-expanding features and improved anti-migration characteristics of the assembly.

The use of the material chosen for endovascular valve replacement in this assembly represents a unique application of a biocompatible substance. Whether the material is formed of elastomer, sclera, small intestine sub-mucosa (SIS), other mammalian tissue, or other suitable material, the venous stent device of this invention will serve as a substitute for deteriorated venous valves which have been altered by thrombosis or congenital hypoplasia. The valve prosthesis within the self-expanding stent will be percutaneously introduced with a small sized catheter delivery system. Justification for development of this invention is based on the incidence of venous disorders that lack adequate endovascular therapy. Patients who are treated surgically undergo a more invasive method that involves greater costs and more numerous potential complications. The minimally invasive technique of this invention will decrease length of hospital stay, lower over-all costs and permit an almost immediate return to normal activity. Indeed, it is believed that the availability of this treatment will dramatically alter the lives of many people, including those who might not have been able to undergo previous surgical techniques for the repair or replacement of damaged venous valves.

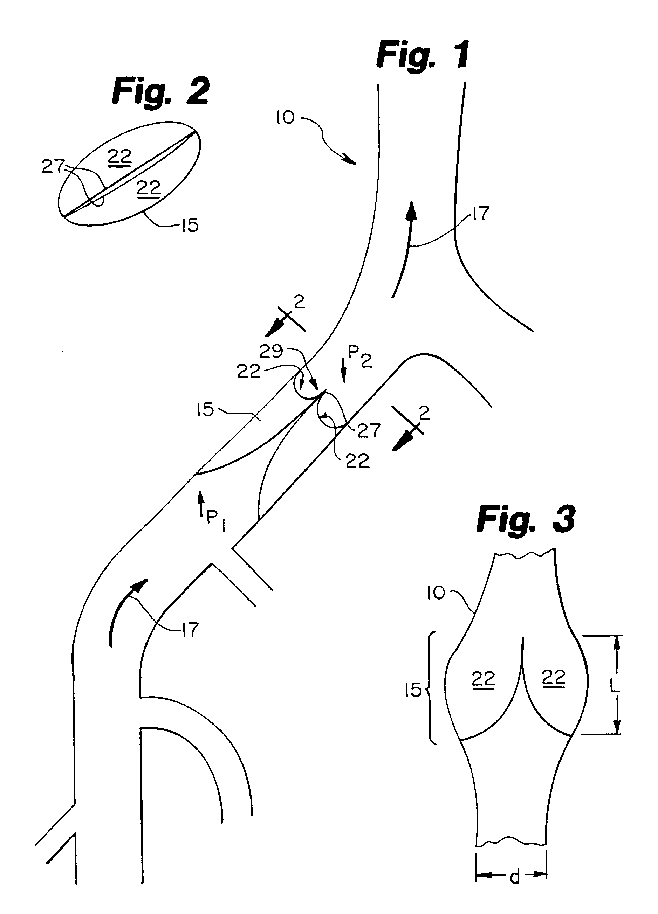

Figure 1 is a schematic representation of an exemplary portion 10 of a human venous system. In venous system portion 10, a representative venous valve 15 is illustrated and shown in a closed position. As is well understood, the flow of blood through venous system 10 is in the direction of arrows 17, with the dominant pressure illustrated by a symbol P₁. Although the venous system is designed to ensure flow of blood from extremities back to the heart, Figure 1 also illustrates the phenomenon of retrograde flow and retrograde pressure which exists in the venous system and which is illustrated by symbol P₂. The design of competent human venous valves takes into account this retrograde pressure. Accordingly, the configuration of bicuspid venous valve 15 accommodates the pooling of the blood at a plurality of sites each known as a valvular sinus 22. The temporal pooling of blood in each sinus or pocket creates retrograde pressure against the valve leaflets and facilitates closure of the free borders 27 of the valve cusp. Although the clear majority of human venous valves are of the bicuspid variety, it is noted that certain venous valve formations in humans may also include other than bicuspid configurations.

Figure 2 is a sectional view taken along line 2-2 of Figure 1. In Figure 2 it may be seen that the free borders 27 of cusp 29 of valve 15 are essentially closed, and are facilitated in maintaining that closure by the pressure of blood pooling in the valvular sinus areas 22. It is recognized that the free borders 27 of the valve cusp may actually present as an undulating shape rather than merely a substantially straight shape across the diameter of the valve when viewed from section 2-2.

As shown in the healthy venous valve schematically represented in Figure 3, the vertical length L of valve 15 cusp 29 is often at least about twice the diameter d of the respective blood vessel. This relationship, though not absolute, is quite common. Also, the free borders 27 of the valvular cusps of bicuspid valve 15, when closed, may contact each other over a length corresponding to approximately 1/5 to 1/2 of the venous diameter d at the site of the particular valve. Thus, the natural human bicuspid venous valve, in a competent state, utilizes both the axial and retrograde pressure of the blood in the valvular sinus, as well as the contact of the lengthy free ends of the valve cusps to maintain closure. In other words, the contact of the free ends is further enhanced by the axial pressure created by the weight and volume of the pooled blood in the sinus areas.

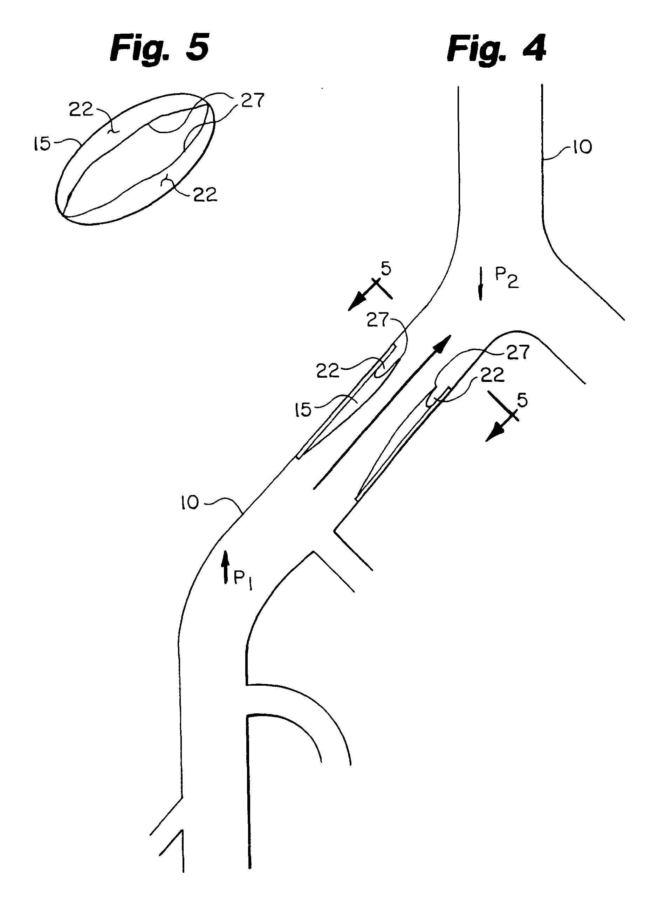

Replication of this phenomenon has generally been beyond the technical ability of known devices or prostheses. The challenge is particularly formidable in view of the anatomy of the venous valve system and in particular the nature of veins themselves. One example of the challenge attendant to venous valve replacement relates to the shape of the veins in the venous system. Indeed, inside the body, veins will have cross-sections of elliptic shape, particularly at the venous valve locations. This is due to the interaction of the skin, the subcutaneous fascia, and other tissue that presses the veins toward the muscles, or the muscles pressing the veins toward the bone. This results in the free ends of the valvular cusps being generally aligned along the longitudinal axis of the above-described ellipse. Therefore, proper insertion of or repair to venous valves involves precise orientation within the vessel. As appreciated from the above description, the optimum apposition of the free ends of venous valve cusps is achieved when the valvular cusps are aligned with the longest diameter of the ellipse. The venous system also includes, as shown in Figure 3, a slight thickening of the vessel wall proximate each venous valve. Figure 4 illustrates venous system portion 10, corresponding to that shown in Figure 1, but with venous valve 15 in an open configuration and normal blood flow proceeding through the valve. Figure 5 illustrates, similar to Figure 2, the action of the free ends 27 of valve 15 cusps.

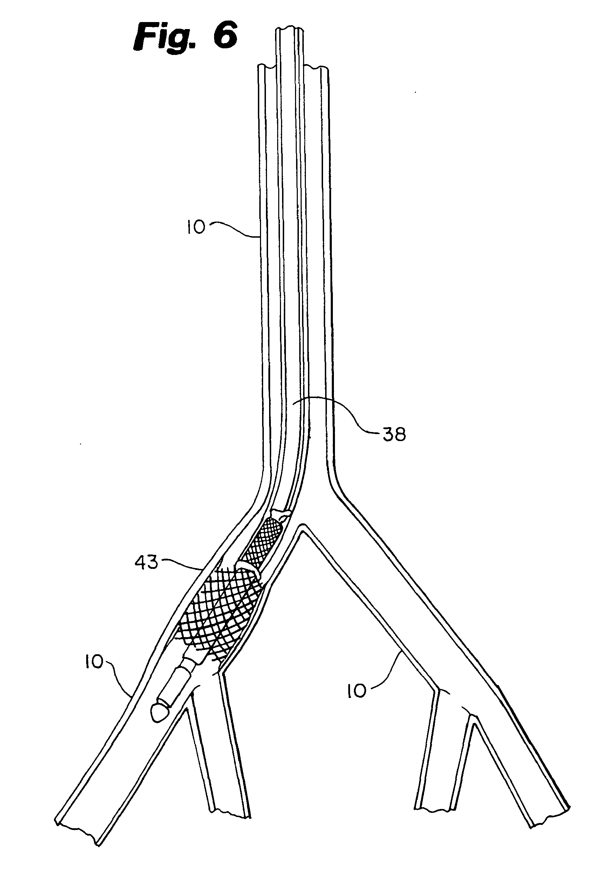

Figure 6 illustrates one embodiment of a deployment technique for deploying a valve and stent into a venous system according to the invention. In this figure, catheter means 38 comprises a portion of an interventional system facilitating, through various guiding technologies, placement and deployment of a stent and valve device 43 at an optimum location within representative venous system 10. It is understood that the optimum location for placement of stent and valve device 43 is generally proximate to existing sites of venous valves in the patient receiving the stent and valve device. However, it is recognized that by using the teachings of this invention it is possible to further optimize and possibly customize a stent and valve device 43 suitable for placement at various locations according to the anatomy of the patient's vein at the specific locations. Further discussion of this feature of the invention is included below. Figure 6 illustrates the stent and valve device 43, with the stent portion partially deployed from the catheter means 38.

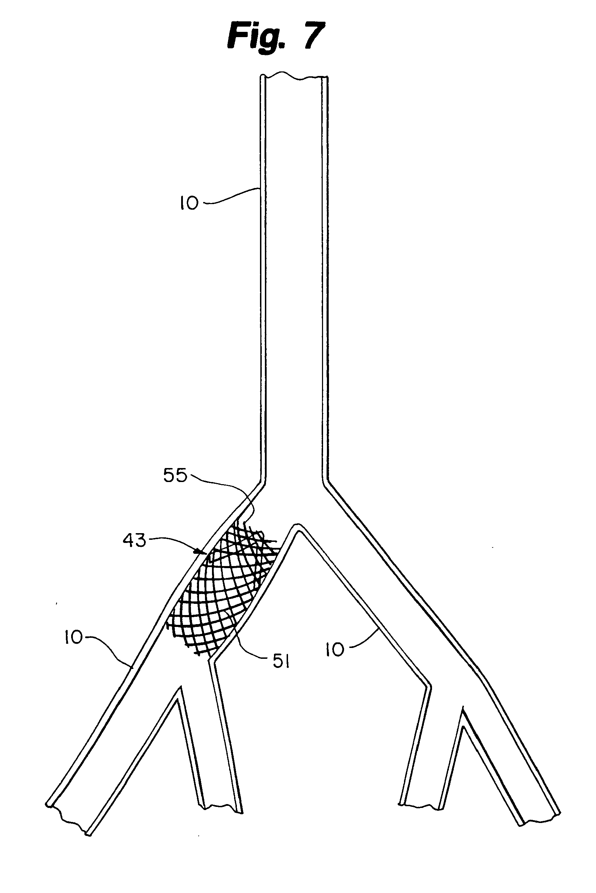

Figure 7 is a representative, schematic, illustration of a venous portion 10, as shown in Figure 6, with a fully deployed stent and valve device 43 therein. In this embodiment, the stent portion 51 of stent and valve device 43 comprises a functionally unitary mesh-type construction. As is understood in the art, stent material may vary according to the lumen or other tissue structure for which it is designed to provide support. In this instance, stent portion 51 accommodates the inner lumen of venous portion 10 sufficient to allow valve portion 55 sufficient diameter to properly function as an artificial venous valve. In Figure 7, valve portion 55 is shown in a closed position. However, the inventors have discovered certain optimal features and properties for stent and valve device 43, which although they may vary according to design and patient need, may represent further improvements over the embodiment illustrated in Figure 7.

The size of a preferred stent and valve device 43 is determined primarily by the diameter of the vessel lumen (preferably for a healthy valve/lumen combination) at the intended implant site, as well as the desired length of the overall stent and valve device. This latter feature is for optimum placement by achieving the best stability during the employment. Thus, an initial assessment of the location of the natural venous valves in the patient is determinative of several aspects of the prosthetic design. For example, the location will determine the number of support struts, the type of valve material selected, the size of deployment vehicle (French size of catheter or other deployment means) and the characteristics of the valvular sinus-like pockets. These and other factors must be considered according to the patient need. In one embodiment, the inventors have utilized algorithmic means for determining proper fit and customization of valves suitable for replacement of incompetent or insufficient valves in the patient. Once again, further discussion of this method is discussed herein below.

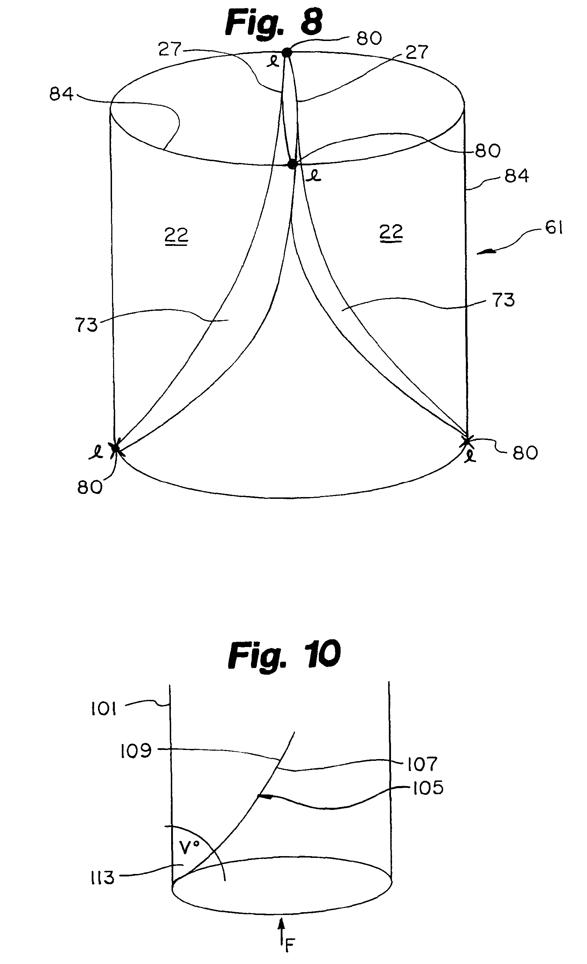

Another representative stent and valve device is shown in Figure 8. In this embodiment, the stent and valve device 61 is simplified to demonstrate the 4-point connection of the selected valve material 73 at connection sites 80 on stent frame 84. Once again, stent frame 84 is shown in very simplified form but is adequate to demonstrate the challenge of having only a very minimum number of connection sites 80. This is challenging because it is important that the valvular sinuses retain the blood above the valve when the valve is in the closed position. Otherwise, a condition known as reflux exists. Obviously, a single point connection to the stent frame portion adjacent the lumenal wall probably will not provide adequate sealing of the valve material to the wall to prevent retrograde flow of blood past the valve. Indeed, what has been determined is the need for multiple point connection of the valve material to the stent structure to properly emulate the natural competent valve.

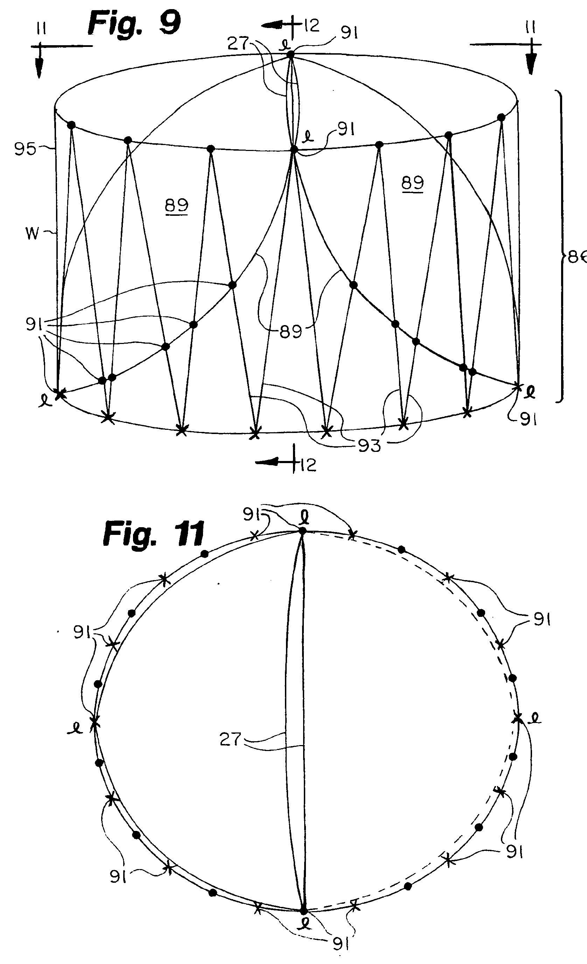

Referring to Figures 9 and 10, an exemplary single-stage stent and valve device, referred to in this embodiment as device 86, comprises multiple connection points 91 for the selected valve material 89 along various struts 93 of stent frame structure 95. The number of struts may vary between merely several struts to upwards of eight to ten struts or even more, as appropriate, according to the lumen size of the vein. For example, in the embodiment of Figure 9, using valve material comprising either naturally occurring sclera tissue or naturally occurring small intestine sub-mucosa (SIS) or other comparable materials, or a combination thereof, it is possible to utilize between about six to twelve struts and deploy the stent and valve device 86 utilizing an approximately ten to fourteen French deployment catheter system.

Another consideration in the design and construction of stent and valve device 86 relates to the angle at which the valve material extends from the circumferential wall, i.e., the inner venous wall. In Figure 10, a partial stent frame structure is shown as a vertical wall strut 101 corresponding to the elastic membrane and endothelial cells of the inner wall of a venous blood vessel. Valve material 105 is shown extending from a portion of strut 101 with a first side 107 corresponding to the lumenal part facing the lumen of the vessel and a parietal part 109 facing the wall of the vessel. Thus, the angle formed between strut 101 (corresponding to the venous wall) and valve material 105 is defined as angle V as shown in Figure 10. The normal flow of blood through the stent and valve device 86 in the embodiment depicted in Figure 10 is in the direction of arrow F. Thus the angle V corresponds to the angle at which the venous valve structure extends from the lumenal wall of a natural venous valve. Although various connection angles occur, it is believed that in the region of the natural valvular agger connection area (corresponding to area 113 of Figure 10) angle V is in a range of between about 35° to 70°. It should also be recognized that the lumenal part of a natural venous valve in a human patient comprises a plurality of crypt-like crevices that further provide means for capturing and collecting the blood pooling in the valvular sinus areas. These crypts do not occur on the parietal side of the valve. Thus, in addition to whatever angle is selected for an artificially manufactured venous valve, it is important to note that there is no disclosure in any known prior artificial valve system to accommodate the angle V and the crypt structure. However, to the extent that a naturally occurring and non-thrombolytic substance may be used for valve material, it is possible that the structure may include substructures that act similar to the collection features of the naturally occurring crypts. For example, if valve material 105 is manufactured utilizing natural tissue such as the above-referenced SIS or sclera tissue, rather than a plastic or elastomer material, then the increased benefits of the tissue structure acting as pseudo-crypts may in fact provide unrealized advantages in a venous valve structure. It should also be appreciated that such advantage may be more accurately emulated subject to the cost limitations and manufacturing techniques attendant to manufacture of inventions disclosed herein. It is worth noting that this and other features of the invention may also be appropriate for placement into a non-venous valve device. Figure 11 illustrates a top plan view of Figure 9, in which the points of attachment are indicated and the free ends 27 of the valve material cusps are shown in apposition.

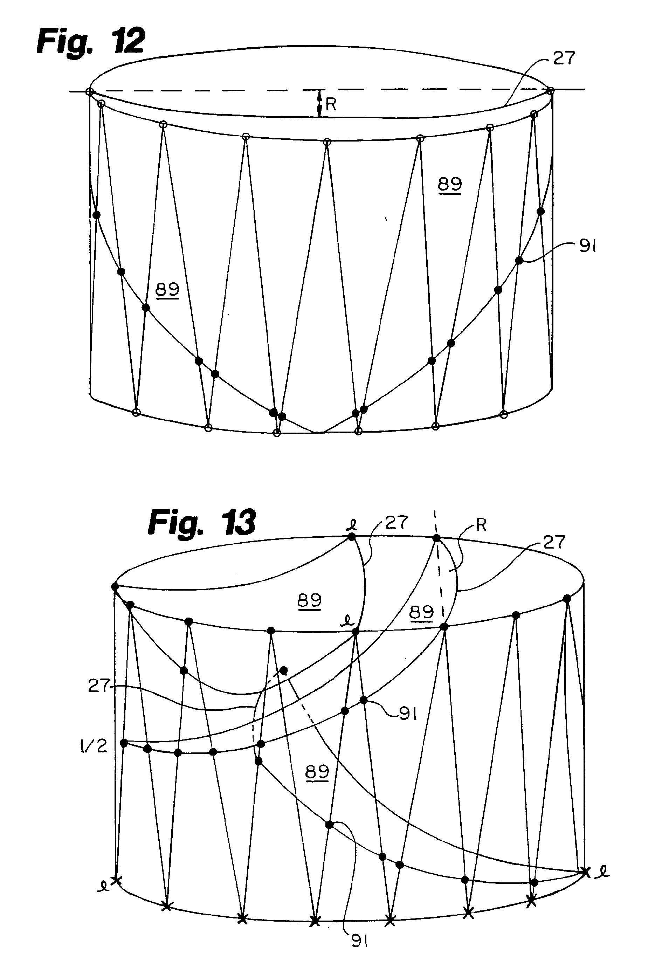

Figures 12 and 13 illustrate the optional radius R which may be formed at the free ends 27 of the valve material 89. A certain amount of radius allows improved functionality for a valve and stent device, subject to the size of the device and the location of use. Figure 13 also indicates several options for attachment locations for free ends 27 on stent frame members. Any of these options may be selected, although a preferred embodiment may also be selected from other figures herein. It is noted that for certain uses valvular sinuses may be either deep or shallow, and the free ends of the valve material may be either centered or offset from a diameter when attached to the stent frame struts or other structure.

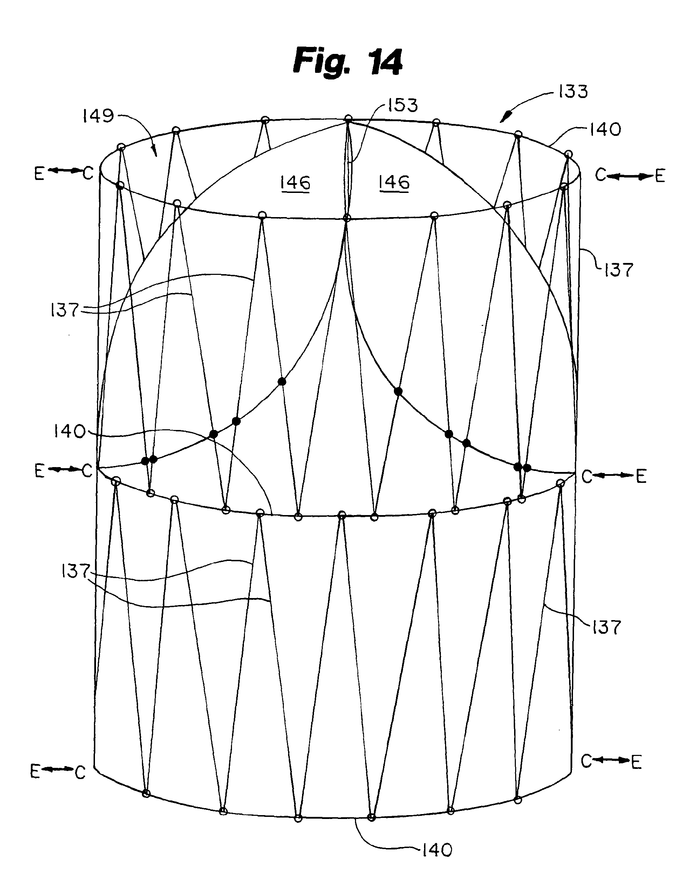

Figure 14 illustrates another embodiment of stent and valve device 133 of the invention. The inventors realized that during deployment, under certain conditions, the self-expanding frame structure 137 and marginal retaining members 140 are inadequate to prevent momentary lack of control. As shown, frame structure 137 will expand and contract according to the pressure applied to the frame in axial directions, as shown by symbols E and C in Figure 14. In particular, when a single stack device is allowed to exit or otherwise be liberated from a deployment means, the device may expand at an undesired rate. This may result in lack of stability during and after deployment. In order to overcome this concern, a double stacked device 133 is provided. As shown, device 133 is configured with valve material 146 arranged so that free ends 153 are proximate an end 149 of the device, rather than lower within the volume of the device. As noted in relation to Figure 13, it is possible within the scope of this invention to alter the location of the valve material, as appropriate. The double stack feature of this device allows for deployment of one stack, and engagement and stability of the deployed stack to occur prior to liberating the second stack. However, the second stack is held is place pre-liberation by the deployment means, e.g. a catheter deployment means.

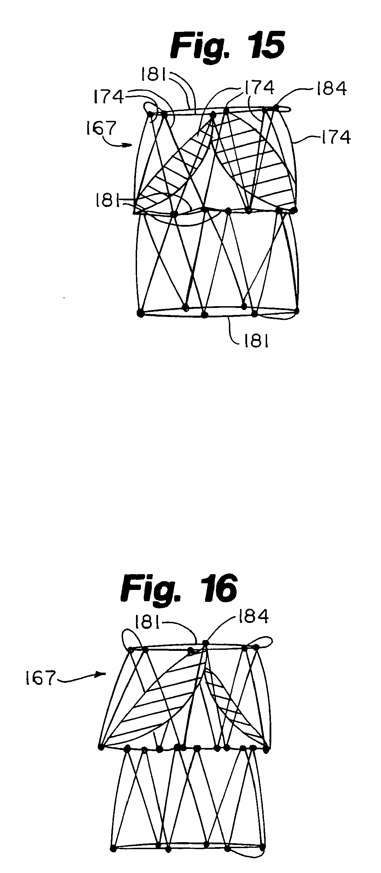

Figures 15 and 16 illustrate further embodiments of a stent and valve device 167, similar to that shown in Figure 14, but having only six struts 174 per stack or stage. These devices are configured with marginal wires or other thin retaining means 181 providing connection through eye-loops 184 on each strut. The truncated cone arrangement of Figure 16 may be particularly useful in certain geometries of vein locations. Figures 15 and 16 each disclose an excellent embodiment for employment as a modular design for controlled deployment. Indeed, such a design as shown in Figure 15 has been tested in vivo, with excellent results for stability and valve operation.

### Example 1

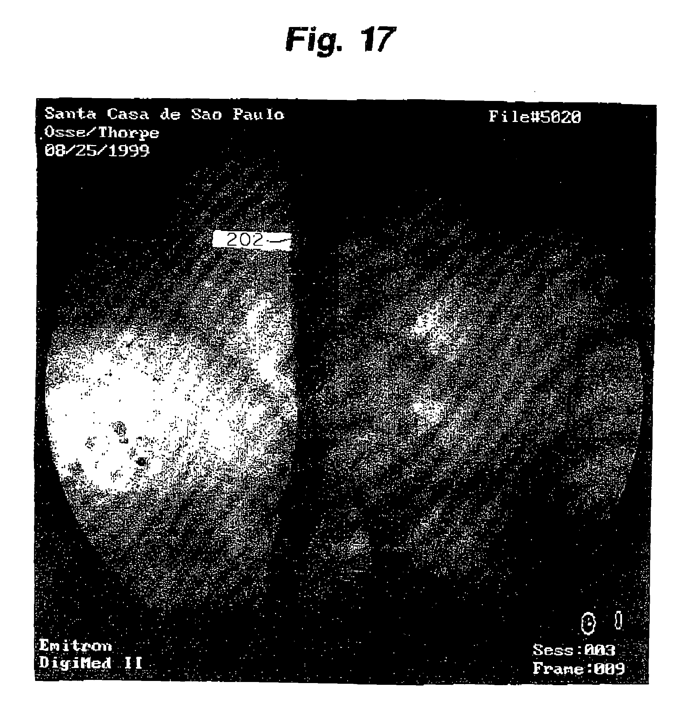

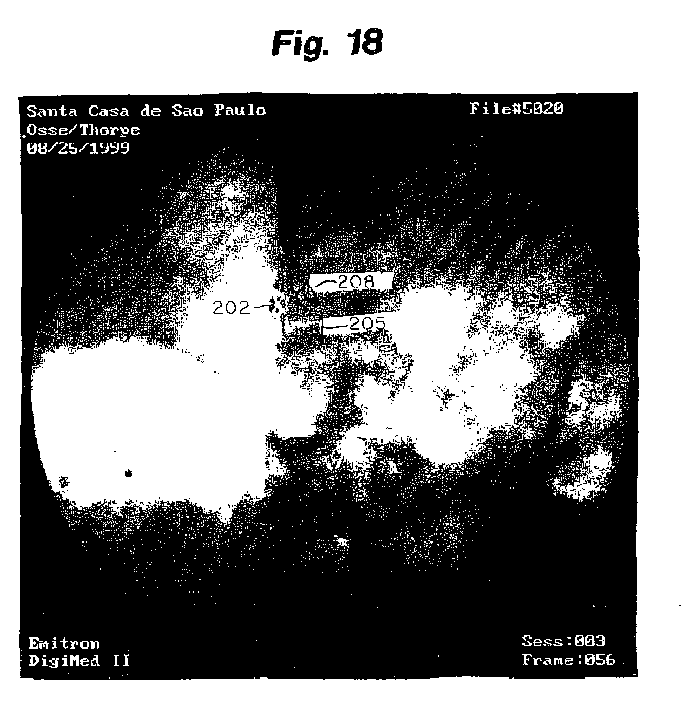

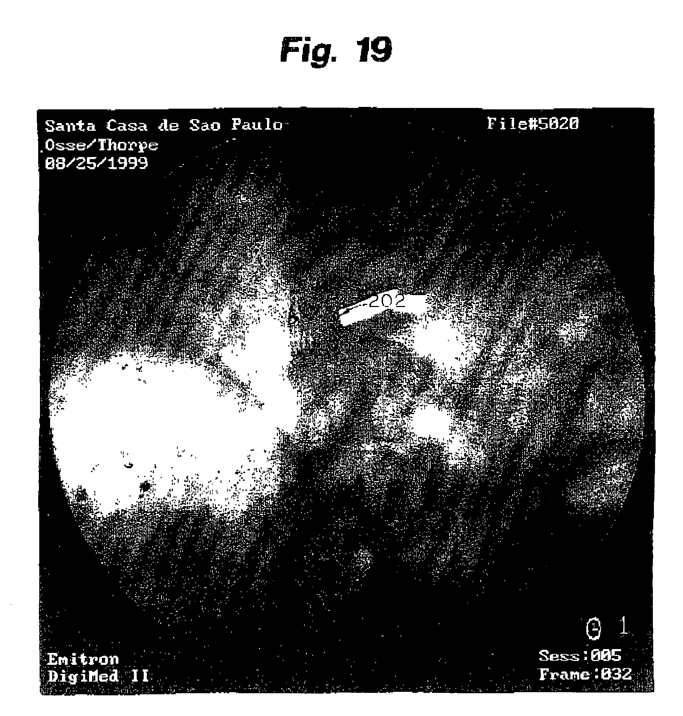

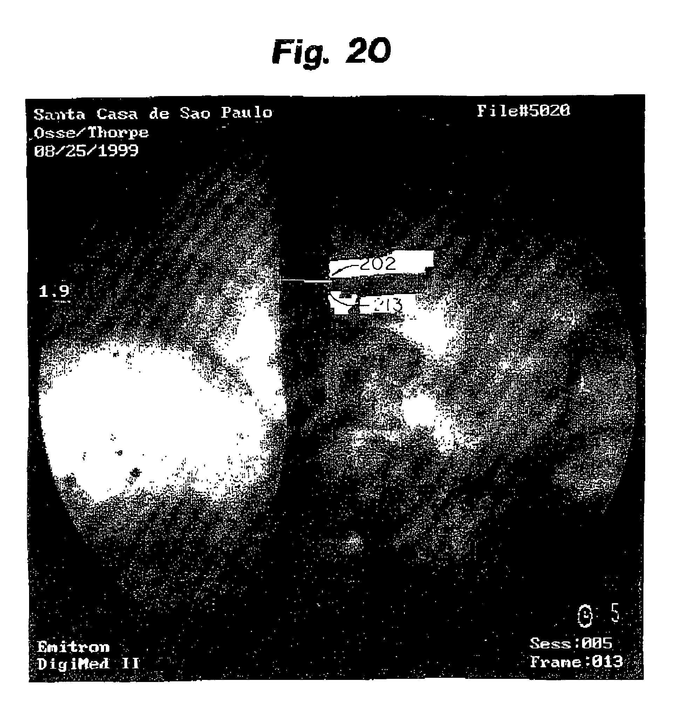

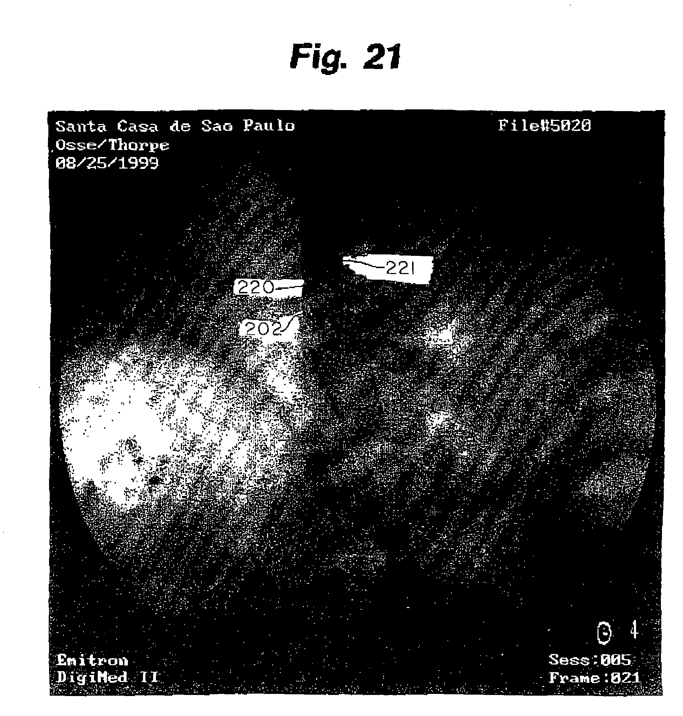

Figure 17 is an in vivo photo image taken of porcine subject #5020 with the Emitron Corporation DigiMed II™ imaging system of a venous system portion in which a device according to the invention is being deployed. Stent and valve device 202 is shown in its compressed configuration within the deployment catheter. Device 202 is approximately 2 cm in length, and is about 15 mm in fully extended diameter. In this example, valve material comprising SIS is used, although sclera was used successfully in similar trials. Figure 18 shows device 202 having deployed first stage 205 to establish a stable platform, and second stage 208 (with the valve material therein) in the process of deployment. Figure 19 shows the fully expanded device 202 which has accommodated the internal lumen of the venous site and has placed the valve material in position. Figure 20 is a further view of device 202 during the systolic flow of blood through the device 202, and with the imaging system measuring gage 213 shown in a verification mode to ensure proper deployment. Verification of valve functionality is also shown in Figure 21. In that Figure, the venous portion is shown in diastole, with the blood pooled in valvular sinus areas 220 and 221 (partially hidden due to orientation of image). Figure 21 clearly illustrates the anti-retrograde feature of device 202 according to several of the teachings of the invention.

### Example 2

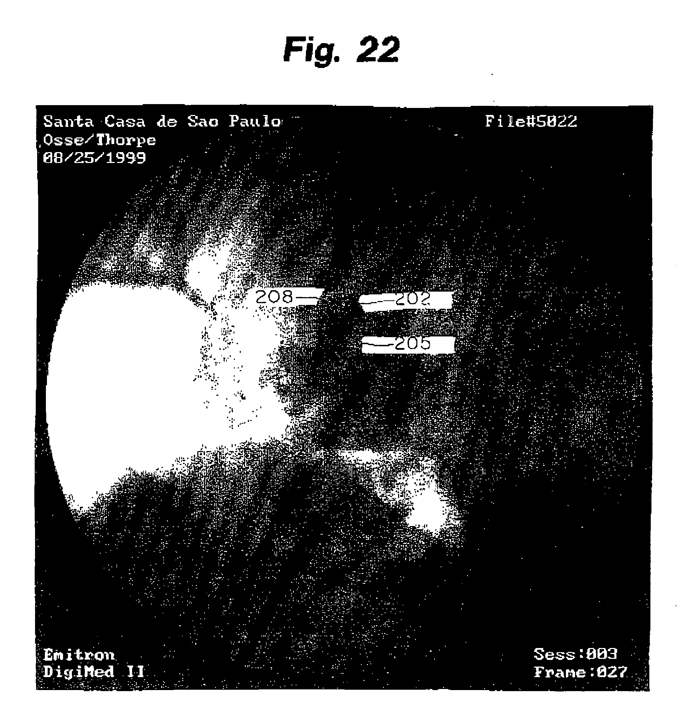

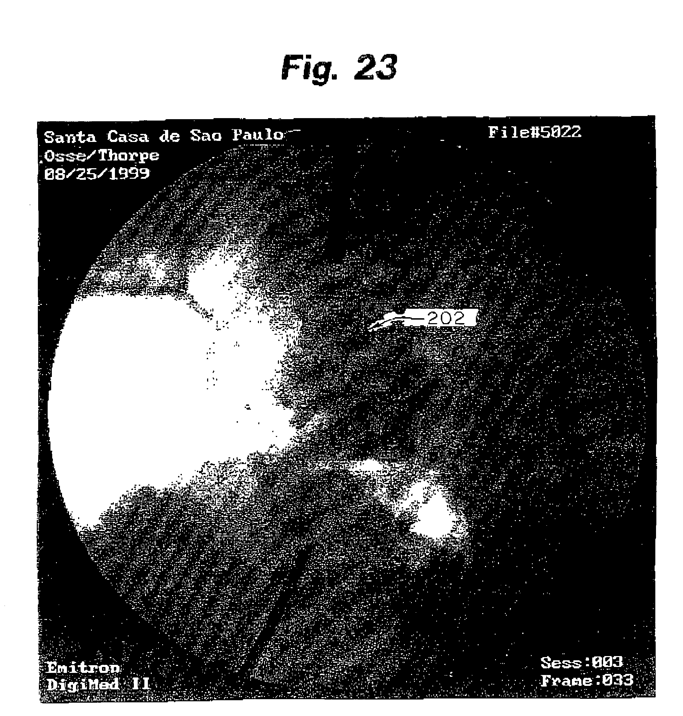

Figure 22 is an in vivo photo image taken of porcine subject #5022 with the Emitron Corporation DigiMed II™ imaging system of a venous system portion in which a device according to the invention is being deployed. Stent and valve device 202 is shown in its partially deployed configuration within the deployment catheter. Device 202 is approximately 2 cm in length, and is about 15 mm in fully extended diameter. In this example, valve material comprising SIS is used, although sclera was used successfully in similar trials. Figure 22 shows device 202 having deployed first stage 205 to establish a stable platform, and second stage 208 (with the valve material therein) in the process of deployment. Figure 23 shows the fully expanded device 202 which has accommodated the internal lumen of the venous site and has placed the valve material in position. Verification of valve functionality was demonstrated in similar manner to that shown in Figures 20 and 21 of Example 1.

### Example 3

The feasibility of a stent-valve combination was studied in the laboratory and in a porcine model. A modified self-expanding stent was combined with a biocompatible material to assess the efficacy, thrombogenicity and histocompatibility of a new prosthesis. The material was configured in a spherical shape and fashioned into adjacent leaflets as a bi-valve design. Leaflets were secured to the stent with 7-0 nylon interrupted sutures. Hydrodynamic and barometric tests were conducted in clear tubular apparatus with variable pulsatile flow. Upon confirmation of valvular integrity, a pilot animal study was conducted. Under general anesthesia, prostheses having a trade name of Valvestent™ were implanted, from a jugular approach, in the distal IVC of 4 six-month old swine. Animals were maintained on warfarin anticoagulant to reduce the risk of embolism.

Following a 30-day observation, with no mortality or extremity edema, a second set of 14 swine underwent baseline phlebography and Valvestent™ prosthesis placement. Follow-up studies were performed at 30, 60 and 180 days consist of phlebography, perfusion retrieval of IVC and iliac veins for histological analysis, and autopsy examination for pulmonary embolus.

Initial hemodynamic testing revealed 10-20% reflux, which was corrected with design modifications. The valve opens with low pressure and maintains shape with elevated hydrostatic pressure above. All animals rapidly recovered from the implantation procedure with no ill effects. Thirty-day mortality is 78% (14/18). One animal died of malignant hyperthermia during surgery, and three animals died at 6-8 days due to internal bleeding related to prolonged prothrombine time. Primary patency of the prostheses at 30 days is 100%. One pilot stent migrated to the pulmonary artery, but remained patent.

The combination of a self-expanding stent and biocompatible material suitable for formation of durable, flexible and non-thrombogenic valve substitute, which does not reflux, appears feasible. Percutaneous delivery of such a Valvestent™ prosthesis assembly would permit a minimally invasive treatment for lower extremity valvular insufficiency.

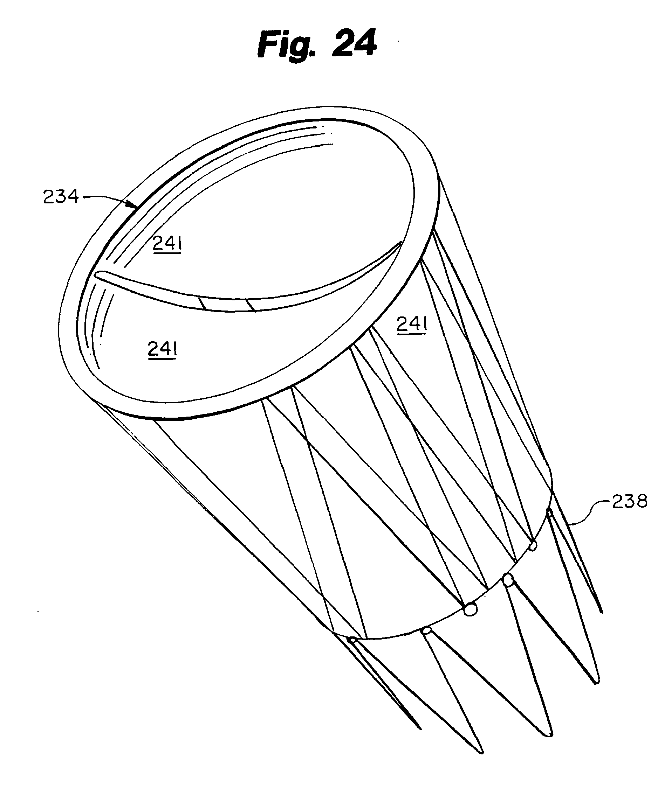

Figure 24 illustrates an alternate embodiment stent and valve device 234. Device 234 has a two stage stent 238 configuration, with valve material 241 arranged both inside the lumen and outside the structure of the generally tubular shaped device. This example is of a relatively shallow sinus variety, and may be one of several embodiments which have dual application to both venous and other vascular uses, including, e.g., an arterial-venous fistula treatment device.

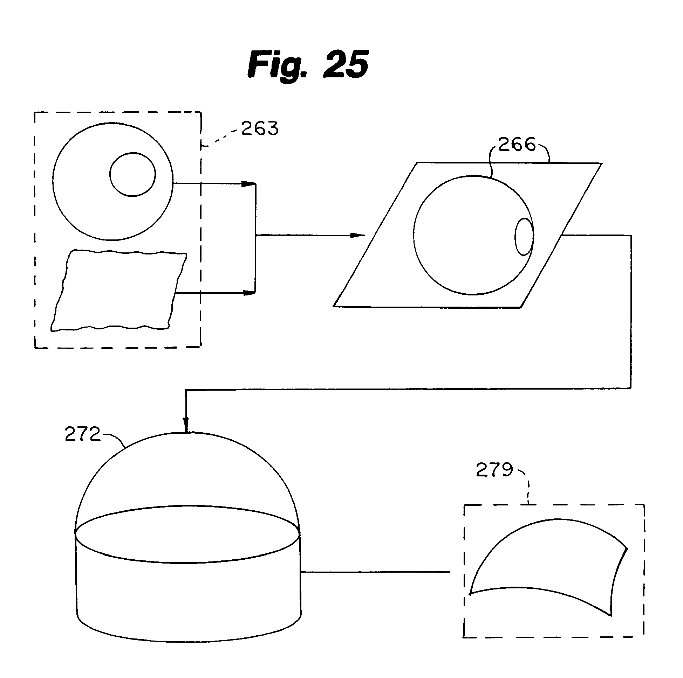

Figure 25 is a flow diagram of a method of configuring a sheet or other portion of valve material for use in stent and valve devices according to the various embodiments of this invention. Block 263 illustrates obtaining basic tissue or other suitable material for use as valve material and providing it in a gently planar form 266 for later processing. In block 272, the material is further shaped over convex/concave shaping means to provide optimum concavity for use in the appropriately sized and shaped valvular sinus configuration. The final shaping and cutting is performed in block 279 at which the precise shape for use in a valve material leaflet is accomplished, including a plurality of arcuate and possibly other edge portions. As disclosed herein, various forms of sclera may be used in the embodiments of this invention. It has excellent features in most respects and is readily harvested at very low cost. Also discussed herein is the use of the known material made of small intestine sub-mucosa, also referred to as SIS. Examples of this material, though not in this use and application, are found in United States Patents No. 4,902,508, 4,956,178, 5,516,533 and 5,641,518, which discuss the teaching of SIS related manufacture and principles of use.

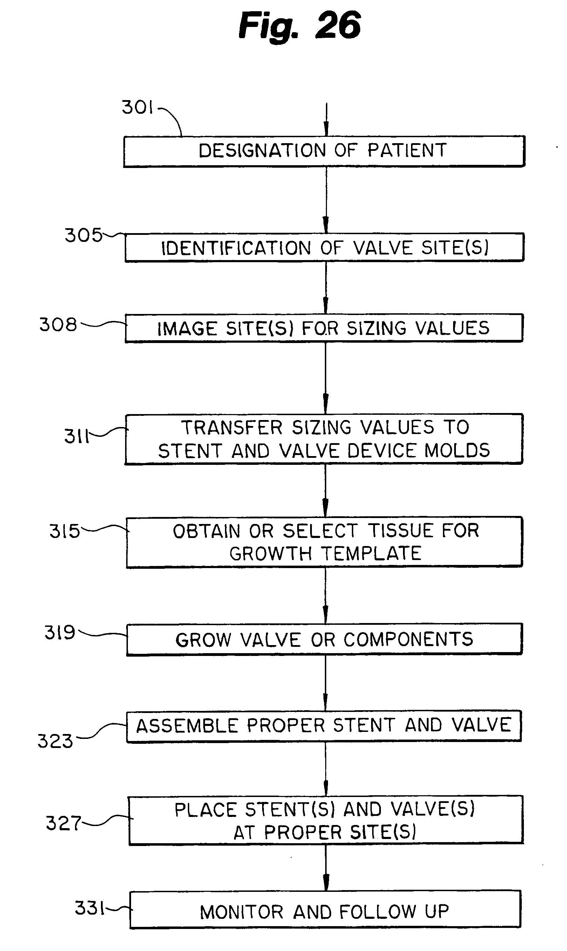

Figure 26 illustrates an optional technique of manufacturing the proper stent and valve device of this invention according to its intended placement in a specific patient. In this technique, it is possible to utilize either some or all steps. In a full utilization of this methodology, a patient is designated 301 for sizing. The insufficient or incompetent valve site or sites are identified 305 using imaging means, such as that identified herein or other systems having highly accurate capabilities. Sizing values for optimum stent and valve configurations are obtained 308 using the imaging means, and the values are then either stoned or otherwise transferred 311 to stent and valve device manufacturing means. Molds or other tools may be effectively utilized in this process. In order to further customize or render more effective in some manner the manufacture of the valve material, it is desired to either select or obtain 315 a tissue sample from the patient or an appropriate subject. The tissue sample may then be utilized in known manner to construct or grow 319 a customized valve portion or portions for later use by the designated patient. Teaching examples of this tissue engineering technology are found in United States Patent Nos. 4,996,154, 5326,357.

Following proper growth of the valve material, the material is then assembled 323 with a properly sized stent, and then placed 327 in the patient at the specifically targeted site, A regimen of monitoring and follow up 331 continues as appropriate. It is believed that the teachings of this method of manufacture and use of the devices herein will greatly facilitate the treatment of many people for a medical problem of great severity and which little history of remedy.

Because numerous modifications may be made of this invention without departing therefrom, the scope of the invention is not to be limited to the embodiments illustrated and described. Rather, the scope of the invention is to be determined by appended claims and their equivalents.

## Claims

1. A replacement valve assembly (133) which is configured for implantation within a venous lumen, the valve assembly including a self-expanding stent (137) and a plurality of flexible members (146), each flexible member (146) conformed to cooperate with at least one other flexible member (146) to unidirectionally admit vascular fluid through the valve assembly (133) and to prevent retrograde flow of the vascular fluid through the valve assembly (133), the stent (137) includes at least two stacks joined so that at least one end of each stack is directly interconnected in series to an end of another stack **characterized in that** at least the two stacks are joined for allowing deployment of one stack prior to deployment of said another stack.

2. The assembly of claim 1, wherein at least a portion of one of said flexible members (146) includes either sclera or small intestine sub-mucosa material.

3. The assembly of claim 1 or claim 2, in which the stent (137) includes flexible and resilient struts (174) configured for use in a venous lumen and which support the flexible members (146).

4. The assembly of claim 3, in which the struts (174) are manufactured with a resilient metallic material.

5. The assembly of claim 4, in which the struts (174) material is selected from either nitinol or stainless steel.

6. The assembly of claim 3, in which the struts (174) are manufactured from a biodegradable-like material designed to dissolve in a patient after a certain period of time.

7. The assembly of any one of claims 3 to 6, in which the flexible members (146) are cusps of a valve having edge portions configured for attachment to the struts (174), and edge portions configured to form free ends (153) that selectively form an opening through the valve assembly (133) or an obstruction in the valve assembly (133).

8. The assembly of any one of the preceding claims, in which the flexible members (146) are bicusps.

9. The assembly of any one of claims 1 to 7, in which the flexible members (148) are generally semi-elliptical in shape.

10. The assembly of any one of the preceding claims, shaped as a generally tubular flexible member (137) conformed to be implanted in a venous lumen in a fixed location.

11. The assembly of claim 3, in which the struts (174) are connected to form a tubular shape and are flexible in a direction generally transverse to a longitudinal axis of the tubular shape, the flexible members (146) being operably disposed in the tubular member.

12. The assembly of claim 11, in which each flexible member (146) defines a first edge portion conformable to said tubular member (137) and a second edge portion, the second edge portion of each flexible member (146) cooperating to enable said unidirectional flow by forming a plurality of free ends (153) which selectively engage and disengage each other.

13. The assembly of claim 3, wherein each stage of the stent includes at least six struts (174).

14. The assembly of claim 13, in which the total length of the assembly (133) is between about 1 and 2cm, and the diameter of any stage is between about 8-20 mm.

15. The method of making the replacement valve assembly (133) of any of the previous claims for implantation, the method comprising the steps of:
providing a flexible biocompatible material;
constructing a plurality of flexible members (146) from the flexible material; and
disposing the flexible members in a tubular member (137) having at least two stacks joined so that at least one end of each stack is directly interconnected in series to an end of another stack for allowing deployment of one stack prior to deployment of said another stack and joined so as to function as a directional flow valve.

16. The method of claim 15, in which sclera or SIS material is provided as the flexible material.

## Patentansprüche

1. Ersatzklappenanordnung (133), die für eine Implantation innerhalb eines Venenhohlraumes ausgebildet ist, wobei die Klappenanordnung einen selbstexpandierenden Stent (137) und eine Vielzahl von flexiblen Elementen (146) aufweist, wobei ein jedes flexible Element (146) angepasst ist, um mit mindestens einem weiteren flexiblen Element (146) zusammenzuwirken, um in einer Richtung Gefäßflüssigkeit durch die Klappenanordnung (133) zu lassen, und um einen rückwärtsgerichteten Strom der Gefäßflüssigkeit durch die Klappenanordnung (133) zu verhindern, wobei der Stent (137) mindestens zwei Stapel umfasst, die so verbunden sind, dass mindestens ein Ende eines jeden Stapels direkt in Reihe mit einem Ende eines weiteren Stapels verbunden ist, **dadurch gekennzeichnet, dass** mindestens zwei Stapel verbunden werden, um die Aufrichtung des einen Stapels vor der Aufrichtung des anderen Stapels zu gestatten.

2. Anordnung nach Anspruch 1, bei der mindestens ein Abschnitt von einem der flexiblen Elemente (146) entweder Sklera- oder kleine intestinale Submukosa-Material umfasst.

3. Anordnung nach Anspruch 1 oder Anspruch 2, bei der der Stent (137) flexible und elastische Streben (174) umfasst, die für eine Verwendung in einem Venenhohlraum ausgebildet sind, und die die flexiblen Elemente (146) tragen.

4. Anordnung nach Anspruch 3, bei der die Streben (174) mit einem elastischen metallischen Material gefertigt werden.

5. Anordnung nach Anspruch 4, bei der das Material der Streben (174) aus entweder Nitinol oder nichtrostendem Stahl ausgewählt wird.

6. Anordnung nach Anspruch 3, bei der die Streben (174) aus einem biologisch abbaubarartigen Material gefertigt werden, das ausgelegt ist, um sich in einem Patienten nach einer bestimmten Zeitdauer aufzulösen.

7. Anordnung nach einem der Ansprüche 3 bis 6, bei der die flexiblen Elemente (146) Höcker einer Klappe mit Randabschnitten sind, die für eine Befestigung an den Streben (174) ausgebildet sind, und wobei die Randabschnitte ausgebildet sind, um freie Enden (153) zu bilden, die selektiv eine Öffnung durch die Klappenanordnung (133) oder eine Behinderung in der Klappenanordnung (133) bilden.

8. Anordnung nach einem der vorhergehenden Ansprüche, bei der die flexiblen Elemente (146) Doppelhöcker sind.

9. Anordnung nach einem der Ansprüche 1 bis 7, bei der die flexiblen Elemente (146) in der Form im Allgemeinen halbelliptisch sind.

10. Anordnung nach einem der vorhergehenden Ansprüche, geformt als ein im Allgemeinen rohrartiges, flexibles Element (137), das angepasst ist, damit es in einen Venenhohlraum in einer unveränderlichen Position implantiert wird.

11. Anordnung nach Anspruch 3, bei der die Streben (174) verbunden sind, um eine rohrartige Form zu bilden, und in einer Richtung im Allgemeinen quer zu einer Längsachse der rohrartigen Form flexibel sind, wobei die flexiblen Elemente (146) funktionsfähig im rohrartigen Element angeordnet werden.

12. Anordnung nach Anspruch 11, bei der ein jedes flexible Element (146) einen ersten Randabschnitt, der an das rohrartige Element (137) angepasst werden kann, und einen zweiten Randabschnitt definiert, wobei der zweite Randabschnitt eines jeden flexiblen Elementes (146) zusammenwirkt, um den einseitig gerichteten Strom zu ermöglichen, indem eine Vielzahl von freien Enden (153) gebildet wird, die selektiv miteinander in Eingriff und außer Eingriff kommen.

13. Anordnung nach Anspruch 3, bei der eine jede Stufe des Stents mindestens sechs Streben (174) umfasst.

14. Anordnung nach Anspruch 13, bei der die Gesamtlänge der Anordnung (133) zwischen etwa 1 und 2 cm und der Durchmesser einer jeden Stufe zwischen etwa 8 bis 20 mm liegt.

15. Verfahren zur Herstellung der Ersatzklappenanordnung (133) nach einem der vorhergehenden Ansprüche für eine Implantation, wobei das Verfahren die folgenden Schritte aufweist:
Bereitstellen eines flexiblen biokompatiblen Materials;
Konstruieren einer Vielzahl von flexiblen Elementen (146) aus dem flexiblen Material; und
Anordnen der flexiblen Elemente in einem rohrartigen Element (137), bei dem mindestens zwei Stapel verbunden sind, so dass mindestens ein Ende eines jeden Stapels in Reihe mit einem Ende eines weiteren Stapels direkt verbunden ist, um die Aufrichtung des einen Stapels vor der Aufrichtung des anderen Stapels zu gestatten, und so verbunden, dass es als eine Richtungsströmungsklappe funktioniert.

16. Verfahren nach Anspruch 15, bei dem Sklera- oder kleine intestinale Submukosa(SIS)-Material als das flexible Material bereitgestellt wird.

## Revendications

1. Assemblage de valvule de remplacement (133), destiné à être implanté dans une lumière d'une veine, l'assemblage de valvule englobant un stent auto-expansible (137) et plusieurs éléments flexibles (146), chaque élément flexible (146) étant adapté pour coopérer avec au moins un autre élément flexible (146), en vue d'une admission unidirectionnelle du fluide vasculaire à travers l'assemblage de valvule (133) et en vue d'empêcher un écoulement rétrograde du fluide vasculaire à travers l'assemblage de valvule (133), le stent englobant au moins deux piles reliées de sorte qu'au moins une extrémité de chaque pile est interconnectée directement en série à une extrémité d'une autre pile, **caractérisé en ce qu'**au moins les deux piles sont reliées pour permettre le déploiement d'une pile avant le déploiement de ladite autre pile.

2. Assemblage selon la revendication 1, dans lequel au moins une partie de l'un desdits éléments flexibles (146) englobe du tissu sclérotique ou du tissu de la sous-muqueuse de l'intestin grêle.

3. Assemblage selon les revendications 1 ou 2, dans lequel le stent (137) englobe des entretoises (174) destinées à être utilisées dans une lumière d'une veine, et supportant les élément flexibles (146).

4. Assemblage selon la revendication 3, dans lequel les entretoises (174) sont fabriquées à partir d'un matériau métallique résilient.

5. Assemblage selon la revendication 4, dans lequel le matériau des entretoises (174) est sélectionné dans le groupe constitué de nitinol ou d'acier inoxydable.

6. Assemblage selon la revendication 3, dans lequel les entretoises (174) sont fabriquées à partir d'un matériau du type biodégradable en vue d'une dissolution dans l'organisme d'un patient après une certaine période de temps.

7. Assemblage selon l'une quelconque des revendications 3 ou 6, dans lequel les éléments flexibles (146) sont des cuspides d'une valvule comportant des parties de bordure destinées à être fixées sur les entretoises (174), et des parties de bordure destinées à former des extrémités libres (153), formant sélectivement une ouverture à travers l'assemblage de valvule (133) ou une obstruction dans l'assemblage de valvule (133).

8. Assemblage selon l'une quelconque des revendications précédentes, dans lequel les éléments flexibles (146) sont des bicuspides.

9. Assemblage selon l'une quelconque des revendications 1 à 7, dans lequel les éléments flexibles (146) ont en général une forme semi-elliptique.

10. Assemblage selon l'une quelconque des revendications précédentes, ayant la forme d'un élément flexible généralement tubulaire (137) adapté pour être implanté dans une lumière d'une veine dans un emplacement fixe.

11. Assemblage selon la revendication 3, dans lequel les entretoises (174) sont connectées pour établir une forme tubulaire, et sont flexibles dans une direction généralement transversale à un axe longitudinal de la forme tubulaire, les éléments flexibles (146) étant placés en service dans l'élément tubulaire.

12. Assemblage selon la revendication 11, dans lequel chaque élément flexible (146) définit une première partie de bordure pouvant être adaptée audit élément tubulaire (137), et une deuxième partie de bordure, la deuxième partie de bordure de chaque élément flexible (146) coopérant pour permettre ledit écoulement unidirectionnel en formant plusieurs extrémités libres (153), s'engageant sélectivement les unes dans les autres ou se dégageant les unes des autres.

13. Assemblage selon la revendication 3, dans lequel chaque étage du stent englobe au moins six entretoises (174).

14. Assemblage selon la revendication 13, dans lequel la longueur totale de l'assemblage (133) est comprise entre environ 1 et 2 cm, le diamètre d'un quelconque étage étant compris entre environ 8 et 20 mm.

15. Procédé de fabrication de l'assemblage de valvule de remplacement (133) selon l'une quelconque des revendications précédentes, comprenant les étapes ci-dessous :
fourniture d'un matériau biocompatible flexible ;
construction de plusieurs éléments flexibles (146) à partir du matériau flexible ; et
placement des éléments flexibles dans un élément tubulaire (137), comportant au moins deux piles reliées de sorte qu'au moins une extrémité de chaque pile est directement interconnectée en série à une extrémité d'une autre pile, pour permettre le déploiement d'une pile avant le déploiement de ladite autre pile, et sont reliées de sorte à faire fonction de valvule de direction de l'écoulement.

16. Procédé selon la revendication 15, dans lequel du tissu sclérotique ou du tissu SIS est fourni comme matériau flexible.
